# EUROPEAN PATENT SPECIFICATION

(11) **EP 0 609 394 B1**
(45) Date of publication and mention of the grant of the patent: **22.12.1999**
(21) Application number: 92924139.6
(22) Date of filing: 23.10.1992
(51) Int. Cl.: E06B 9/00, A62C 2/06, A62C 3/06, F42D 5/045, B32B 3/24

(54) **ANTI-EXPLOSION PADS AND THEIR METHOD OF USE**
EXPLOSIONSSCHUTZKISSEN UND DEREN VERWENDUNGSVERFAHREN
MATELAS ANTI-EXPLOSION ET LEUR PROCEDE D'UTILISATION

(30) Priority: 25.10.1991 US 784171
(43) Date of publication of application: 10.08.1994
(73) Proprietor: FIREXX CORPORATION, Riyadh, 11418 (SA)
(72) Inventor: ALHAMAD, Shaikh, Ghaleb, Mohammad, Yassin, Riyadh, 11418 (SA)
(74) Representative: Waxweiler, Jean
(86) International application number: US9209221
(87) International publication number: WO9308361

(56) References cited:
- EP-A- 0 256 239
- FR-A- 2 669 229
- GB-A- 554 562
- US-A- 3 356 256
- US-A- 3 431 818
- US-A- 3 648 613
- US-A- 4 149 649
- US-A- 4 727 789
- US-A- 4 828 932

## Description

### FIELD OF THE INVENTION

This invention relates generally to anti-explosive pads which may be used to protect structures against the disastrous effects of accidental or otherwise unwanted explosions. More particularly, the invention involves stratiform anti-explosion pads including multiple layers of lightweight expanded metal net. The invention also involves the method of applying such pads for taking advantage of their anti-explosive characteristics.

### BACKGROUND

As is well known, the production and use of explosives is an extensive and far-reaching industry. Research through the centuries has developed many useful applications for the known explosives, including the industrial blasting utilized in the mining and road building industries, as well as the harnessing of tiny explosions for use in internal combustion engines. The military use of gun powder and other explosives in rifles, artillery, bombs and the like is also well known.

Concurrently with the benefits derived from the useful application of explosives, the world has been forced to endure the disastrous results which too often occur when explosives are accidentally detonated, such as in the case of explosions in coal mines, fuel tank fields, homes, automobiles, ships, airliners, and the like. Similarly, the world is faced with incidents in which bombs are used for terrorist or other illegal purposes.

There has been a considerable effort to develop products and methods for protecting structures against the destruction which occurs when explosives are detonated in their vicinity, either accidentally or for sinister purposes. Although some progress has been made, the loss of human lives and the destruction of property from explosions continues at an unacceptable rate, and there is continued intense effort to find practical, effective and economical ways of improving anti-explosive products and techniques.

US-A-4 828 932 disclose a porous metallic material comprising a laminate of an expanded metal and a fibrous metallic fiber both of which are pressed to be joined to each other to form a sound absorbing material.

In US-A-4 727 789 there is described a stratiform lightweight anti-explosion pad for protecting against the destructive impact of an explosion according to the preamble of claim 1. More specifically, US-A- 4 727 789 discloses an anti-explosion pad comprising a first sheet of porous metal net, a second sheet of porous metal net spaced apart from the first sheet, and an inner core layer of air-permeable material separating the first and second sheets.

A method for protecting a structure against the destructive impact of an explosive according to the preamble of claim 12 is also known from US-A-4 727 789.

It is an object of the present invention to provide a padding material which possess significantly enhanced explosion suppressing properties.

It is another object of the invention to produce an anti-explosion pad containing extremely lightweight components which serve in a surprisingly effective manner to dissipate the shock waves resulting from the detonation of an explosive material.

It is a further object to provide a method for use of the new anti-explosion pad in the protection of structures which are otherwise subject to severe damage from the explosive force of a detonated bomb.

To achieve this, the anti-explosion pad of the invention is characterized by the features claimed in the characterizing part of claim 1 and the invention provides a method according to the characterzing part of claim 12.

Basically, according to the invention, the first and second sheets of expanded metal net are made from slitted metal foil having a thickness in the range from about 0.028 to 1.0 mm, and the inner core layer is at least 2,54 cm (1 inch) thick.

Preferred embodiments of the anti-explosion pad and its method of manufacture are specified in the dependent claims.

Other features and advantages will become apparent as the specification proceeds.

### SUMMARY OF THE INVENTION

This invention is based on the discovery that walls and other structural elements can be effectively protected against bomb explosions by interposing between them and the bomb a lightweight pad containing multiple sheets of expanded metal net separated by a layer of porous material. It has been found that the presence of the expanded metal net effectively deflects and dissipates the shock waves resulting from the detonation of the explosive material, so that the wall or other structural element maintains its physical integrity in spite of the explosion.

In a preferred embodiment, the pad is retained between front and back covers, and the sheets of expanded metal net are made from slit foil such as a magnesium alloy metal, while the inner core is a porous material such as fiberglass, cotton batting, or an assembly of miniature balls formed from expanded metal net.

The invention also comprises a method for protecting structures against the impact of explosions comprising interposing between said structure and said explosive a stratiform anti-explosion pad of the nature described above.

### BRIEF DESCRIPTION OF THE DRAWINGS

FIG. 1 is a cross-sectional elevation of the anti-explosion pad of the present invention, showing the various component layers.

FIG. 2 is a cross-sectional elevation of an optional variation of the explosion pad of the present invention, showing the inclusion of various additional optional layers.

FIG. 3 is a top view of a slitted metal foil sheet, which can be expanded by stretching to provide the expanded metal net usable in the present invention.

FIGS. 4 through 7 are top views of the expanded metal net, showing the changes in configuration as the slitted sheet is pulled to open up the expanded metal net.

### DETAILED DESCRIPTION OF THE INVENTION

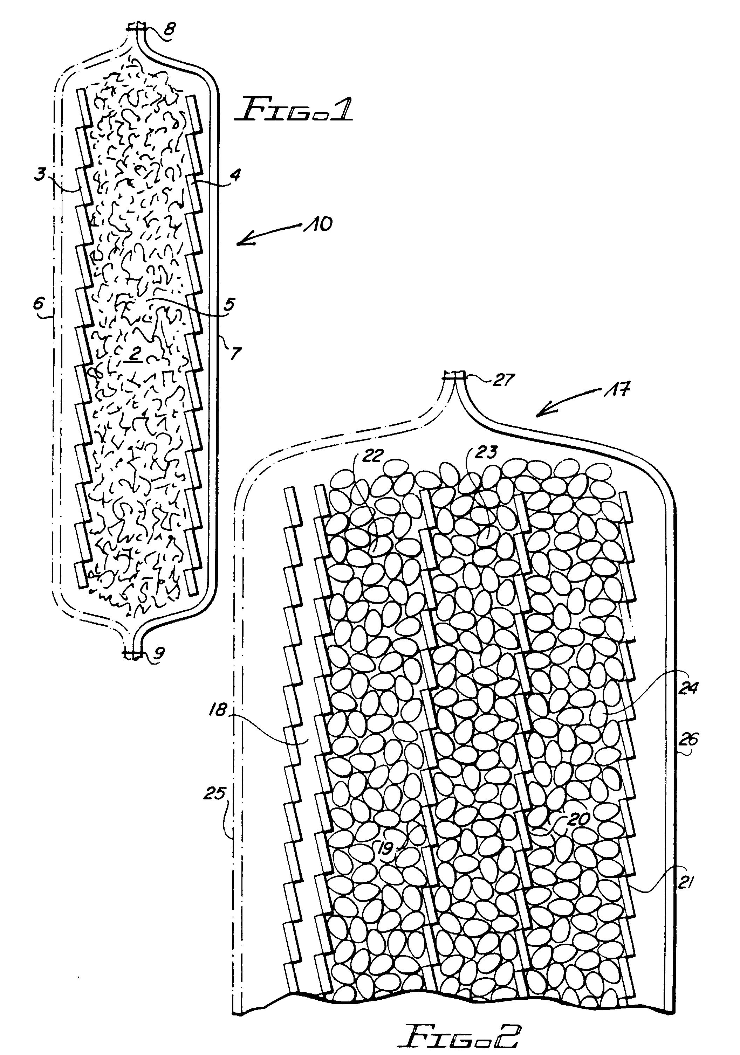

Referring to the drawings, the basic stratiform anti-explosion pad of the present invention is shown in FIG. 1, wherein the pad 10 contains sheets 3 and 4 made of expanded metal net and separated from each other by an inner core 5 made of an air-permeable material. Although not essential to the invention, it is desirable for certain purposes to enclose the above pad between front and back covers 6 and 7 to maintain the integrity of the pad and prevent slipping or shifting of the elements. For this purpose, the front and back covers 6 and 7 may be bound together by stitching, stapling or other known fastening means at seams 8 and 9.

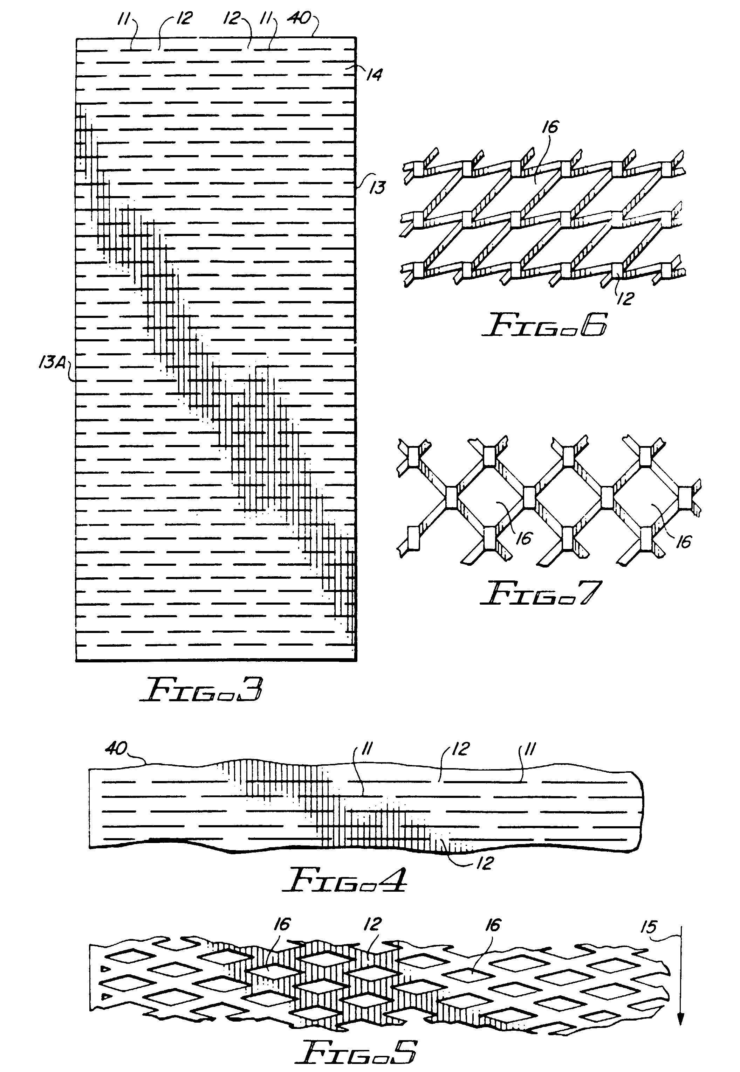

The expanded metal employed in sheets 3 and 4 is formed by slitting a continuous sheet of metal foil in a specialized manner and then stretching the slitted sheet to convert it to an expanded prismatic metal net having a thickness substantially greater than the thickness of the foil. Referring to the drawings, FIG. 3 shows a sheet of metal foil provided with discontinuous slits appropriate for the present invention. The length and width of the sheet may be chosen from any number of practical dimensions, depending on the size of the anti-explosion pad to be produced.

As noted in FIG. 3, sheet 40 is provided with discontinuous slits 11 in spaced apart lines which are parallel to each other but transverse to the longitudinal dimension of the sheet 40. The slits 11 in each line are separated by unslit segments or gaps 12, and it will be noted that the slits 11 in each line are offset from the slits 11 in adjacent lines. Similarly, the gaps 12 in each line are offset from the gaps 12 in adjacent lines. The lines of slits run parallel to the longitudinal edges 13 and 13A of the continuous sheet of metal foil. Apparatus for producing the slitted metal foil is described in detail in US-A- 5 001 017.

When the slitted metal foil as shown in FIG. 3 is stretched by subjecting it to longitudinal tension, it is converted into an expanded metal prismatic net, usable as elements 3 and 4 of FIG. 1 of the present invention. In the stretching procedure, the horizontal surfaces of foil are raised to a vertical position, taking on a honeycomb-like structure. This conversion is shown in FIGS. 4 through 7 of the drawings. The slitted metal foil 10 is shown in FIG. 4 prior to stretching. When longitudinal tension is applied in the direction of arrow 15, the slits 11 begin to open and are converted to eyes 16, and the product assumes the appearance shown in FIG. 5. The application of more tension causes a greater opening of the slits, and the product expands into the honeycomb-like, prismatic form shown in FIG. 6. When even further tension is applied, the configuration reaches its desired end point, as in FIG. 7. The conversion illustrated in FIGS. 4 through 7 is accompanied by an increase in thickness of the product, the final thickness of the honeycomb product being approximately twice the value of the space 14 between each line of slits.

For the anti-explosion pad usage of the present invention, it is desired that the metal foil be very thin and that the slits in each line and the spaces between the lines be very small. Thus, the thickness of the foil used to produce the metal net should be in the range between 0.028 and 1.0 mm, and the preferred thickness is between 0.028 and 0.2 mm. The length of each slit 11 is in the range between 1 and 2.5 cm, and the unslit sections or gaps 12 between each slit are in the range between 2 to 6 mm long. The distance 14 separating lines of slits may be varied, depending on the thickness desired for the resulting expanded metal net. The distance 14 is ordinarily in the range between 1 and 4 mm, so that the thickness of the resulting expanded metal net is normally in the range between about 2 and 8 mm. The preferred value for distance 14 is either 1 mm or 2 mm.

The kind of metal used in the metal foil may be selected from a wide number of metals or alloys which may be produced in the form of a thin foil. For the purposes of the present invention, it is preferred to use alloys of magnesium with certain other compatible substances. Thus, for example, it is desirable to use an alloy of magnesium with substances such as aluminum, copper, zirconium, zinc, strontium, Rn(electron), silicon, titanium, iron, manganese, chromium, and combinations thereof. Alloys such as the above have the valuable characteristic of not only being lightweight, strong, elastic, heat-conductive, etc., but also the important characteristic of being nonflammable. A particularly useful combination is the alloy of magnesium with aluminum and copper. Another preferred combination is the alloy of magnesium with zirconium and strontium. To a somewhat lesser degree, alloys in which aluminum is substituted for the magnesium, are useful in the practice of the invention.

Further advantages are obtained if the expanded metal net is coated with materials such as an alkaline bichromate or an oleate, which are effective in preventing any fire which may be initiated by detonation of the explosive. When heated, these materials emit a dense vapor which envelop the area and assist in preventing the ignition of construction materials in the area.

The inner core layer 5 may be any suitable air-permeable material such as fiberglass, cotton batting, or other similar non-woven substances. A particularly suitable core material for the layer 5 is an assembly of balls formed from expanded metal net. Such balls are most effective when formed in the shape of small ellipsoids. The ellipsoids are produced by cutting expanded metal net sheets (such as shown in FIGS. 3 through 7) into small segments and then mechanically forming them into the ellipsoid shape. The ellipsoids generally have a short diameter in the range of 20 to 30 mm, and a long diameter in the range of 30 to 45 mm. Apparatus for producing the ellipsoids is described in detail in US-A- 5 001 017.

The inner core layer 5 is in the range between 2.54 to 15.24 cm (1 to 6 inches) thick. A thickness less than this provides diminishing protection, and thicknesses above this range, although effective, add bulkiness which is not practical under most conditions.

For certain uses, it is desirable that the layers 3, 4 and 5 be bound together in a cohesive pad by the use of front and back covers 6 and 7, which may be secured at seams 8 and 9. Any suitable material may used for the back cover 7. However, it is essential that the front cover 6 be made of an air-permeable material such as a metal or fiber screen, which will allow the shock and heat waves of the bomb explosion to reach layers of expanded metal net 3 and 4 and allow said layers to diffuse and dissipate the said waves before they reach the structure to be protected. If the front cover 6 is a solid, impermeable material, the shock waves of the detonated explosive will exert their full unattenuated force against the impermeable surface and will destroy not only the protective pad but also the structure intended to be protected. It is essential therefore that the front cover be air-permeable, as indicated, and also that it be placed in position facing the direction from which the explosive forces will originate.

The invention is not limited to the use of only two layers of expanded metal net, separated by a single core layer. For some applications, involving heavier charges of explosives, it is advantageous to employ three or four layers of metal net, separated by matching cores of porous material. It is also useful in some environments to employ two or more sheets of metal net in contact with each other in a single layer.

FIG. 2 illustrates an embodiment of the invention, in which a double layer of expanded metal net is employed adjacent the front surface of the pad and additional layers of metal net, separated by layers of ellipsoid filling material, are laid up behind the front double layer. The extra layers of metal net and spacing material provide enhanced protection against explosions. Referring to FIG. 2, the enhanced stratiform anti-explosion pad 17 contains layers 18, 19, 20 and 21 made of expanded metal net and separated from each other by inner cores 22, 23 and 24 made of an assembly of ellipsoids of the type described above. The front layer 18 is composed of a double layer of expanded metal net. The pad 17 is enclosed between front and back covers 25 and 26, which are bound together by stitching, stapling or other known fastening means such as seam 27. As indicated previously in connection with the embodiment of FIG. 1, it is essential that the front cover 25 be air-permeable and that it be placed facing the direction from which the explosive shock waves will come. Although the cores 22, 23 and 24 are illustrated in the form of ellipsoids, which are preferred, it will be understood that the core material may be any suitable air permeable material such as fiberglass, cotton batting, or other similar non-woven substances. A single pad may, for certain purposes, be made with different core materials in the various core layers, as for example in a pad with core 22 being ellipsoids and the remaining core layers being fiberglass.

Statiform pads of the nature described above provide remarkable protection against the destructive forces of an explosion. Although the proportion of expanded metal net to the overall weight of the structure being protected is very minute (i.e., between .05-1%), the special honeycomb configuration and the heat conductivity of the expanded metal net effectively dissipate the shock waves and thermal effects of a close range bomb explosion. Thus, for example, a concrete block wall covered with the anti-explosion pad of the present invention, suffers no damage from a 0.45 kg (one-pound) TNT bomb detonated 12.7 cm (5 inches) in front of the wall; whereas, without the pad, the wall is obliterated.

The anti-explosion pad may readily be applied to the surface of structures by means of nails, staples, adhesives, and the like. When in place, the invention has widespread applicability for the protection of structures against explosions. Applications in homes and commercial buildings include covering the walls of garages, furnace rooms, or other areas where fuel tanks or other explosive materials are located. In automobiles, the firewall between the engine compartment and the passenger area may be covered with the anti-explosive pad. For anti-terrorist purposes, the walls of airliner luggage compartments may readily be covered with the product of the invention, to contain and suppress the shock and concussion of a bomb and prevent damage to the controls and other vital structural elements of the plane. The material may be fabricated into walking shields for use by police and firemen at risk from bomb explosions.

The following examples describe specific embodiments which illustrate the invention but should not be interpreted as limiting the scope of the invention:

### EXAMPLE 1

A wall 1.83 m (6 feet long), 1.83 cm (6 feet high), and 15.24 cm (6 inches) thick was constructed of concrete block, resting on a 15.24 cm (6 inch) poured concrete footing in the ground. The entire front surface of this wall was covered with a pad having the construction shown in FIG. 1 of the drawings.

The expanded metal net used in the two layers of the pad was made from an alloy comprising 0.25% Si, 0.3% Fe, 0.01% Cu, 0.01% Mn, 10% Al, 0.01% Zn, 0.1% Ti, and the remainder Mg. The metal foil was .1 mm thick, and in its expanded form the metal net was 2 mm thick. The inner core was a 5.08 cm (2 inch) thick layer of fiberglass. The padding material had front and back covers made of metal screening with a mesh of 4 microns.

A 0.45 kg (one pound) bomb of TNT (trinitrotoluene) in a plastic container was placed on the ground 12.7 cm (5 inches) from the covered surface of the wall and detonated. In spite of the extreme impact, the wall remained intact and showed no signs of damage. The front surface of the anti-explosive pad showed only slight scarring.

Following this, the pad was removed from the wall, and a second one pound bomb of TNT in a plastic container was placed on the ground 12.7 cm (5 inches) from the wall and detonated. The wall was obliterated.

### EXAMPLE 2

A wall was built, having the same dimensions, materials and configuration as in Example 1. The wall was covered with an anti-explosion pad having the structure shown in FIG. 2 of the drawings.

The expanded metal net used in the pad was made from an alloy comprising 0.25% Si, 0.3% Fe, 0.01% Cu, 0.01% Mn, 10% Al, 0.01% Zn, 0.1% Ti, and the remainder Mg. The metal foil was .1 mm thick, and in its expanded form the metal net was 2 mm thick. The metal foil was coated with an oleate composition. Each of the inner cores was a 2.54 cm (1 inch) thick assembly of ellipsoids made from the same material as the layers of expanded metal net. The padding material had front and back covers made of metal screening with a mesh of 4 microns.

A 0.9 kg (two pound) bomb of TNT (trinitrotoluene) in a metal shell was placed on the ground 12.7 cm (5 inches) from the covered surface of the wall and detonated. In spite of the extreme impact, the wall remained intact and showed no signs of damage or burning. The front surface of the anti-explosive pad showed only slight scarring.

Although preferred embodiments of the invention have been described herein in detail, it will be understood by those skilled in the art that variations may be made thereto without departing from the scope of the invention as specified in the claims.

## Claims

1. A lightweight anti-explosion pad (10; 17) for protecting against the destructive impact of an explosion,
said pad (10; 17) comprising a first sheet (3;18) of expanded metal net, a second sheet (4;20) of expanded metal net spaced apart from said first sheet (3;18),and an inner core layer (5; 22,23) of air-permeable material separating said first and second sheets,
characterized in that said first and second sheets (3, 4; 18, 20) of expanded metal net are made from slitted metal foil (10) having a thickness in the range from about 0.028 to 1.0 mm, and
in that said inner core layer (5; 22,23) is at least 2,54 cm (1 inch) thick.

2. The anti-explosion pad of claim 1, characterized in that said expanded metal net is made from magnesium alloy foil (40).

3. The anti-explosion pad of claim 2, characterized in that said magnesium alloy foil (4) has a thickness in the range from about 0.028 to 0.5 mm.

4. The anti-explosion pad of claim 1, characterized in that said expanded metal net has a thickness of about 2 to 8 mm in its expanded form.

5. The anti-explosion pad of claim 1, characterized in that said inner core (5, 22, 23) of air-permeable material comprises fiberglass.

6. The anti-explosion pad of claim 1, characterized in that said inner core (5; 22, 23) of air-permeable comprises cotton batting.

7. The anti-explosion pad of claim 1, characterized in that said inner core (22, 23) of air-permeable material comprises an assembly of balls (22, 23) formed from expanded metal net.

8. The anti-explosion pad of claim 1, characterized in that said pad (10; 17) is retained between front and back covers (6, 7; 25, 26), said front cover (6; 25) comprising an air-permeable material.

9. The anti-explosion pad of claim 8, characterized in that said front cover (6; 25) comprises a woven screen.

10. The anti-explosion pad of claim 1, characterized in that said pad (17) has its component layers bound together to form a unified structure containing:
a. a front cover layer (25) formed of an air-permeable material,
b. a first anti-explosion layer comprising said first sheet (18) and at least one further sheet of said expanded metal net laid up in contact with each other and adjacent said front cover layer (25), said further sheet of expanded metal net in said first anti-explosion layer being made from slitted metal foil having a thickness in the range from about 0.028 to 1.0 mm;
c. a second anti-explosion layer comprising at least said second sheet (20) of expanded metal net, said second anti-explosion layer being spaced apart from said first anti-explosion layer;
d. said inner core layer (22, 23) of air permeable material separating said first and second anti-explosion layers; and
e. a back cover layer (26) bound to and cooperating with said front cover layer (25) to provide said unified structure.

11. The anti-explosion pad of claim 10, characterized in comprising a second inner core layer (24) of air-permeable material at least 2, 54 cm (1 inch) thick separating said second anti-explosion layer and said back cover layer (26).

12. A method of protecting a structure against the destructive impact of an explosive, comprising:
interposing between said structure and said explosive a stratiform lightweight anti-explosion pad (10; 17) comprising a first sheet (3; 18) of porous metal net, a second sheet (4; 20) of porous metal net spaced apart from said first sheet (3; 18), and an inner core layer (5; 22, 23) of air-permeable material separating said first and second sheets,
characterized by making said first and second sheet (3, 4; 18, 20) of expanded metal net from slitted metal foil (10) having a thickness in the range from about 0.028 to 1.0 mm,
said inner core layer (5; 22, 23) interposed between said first and second sheets being at least 2,54 cm (1 inch) thick.

13. The method of claim 12, characterized in that said expanded metal net is made from magnesium alloy foil.

14. The method of claim 13, characterized in that said magnesium alloy foil (40) has a thickness in the range of 0.028 to 0.5 mm.

15. The method of claim 12, characterized in that said expanded metal net has a thickness of about 2 to 8 mm in its expanded form.

16. The method of claim 12, characterized in that said inner core (5; 22, 23) of air-permeable material comprises fiberglass.

17. The method of claim 12, characterized in that said inner core (5; 22, 23) of air-permeable material comprises cotton batting.

18. The method of claim 12, characterized in that said inner core (22, 23) of air-permeable material comprises an assembly of balls (22, 23) formed from expanded metal net.

19. The method of claim 12, characterized in that said pad (10; 17) is retained between front and back covers (6,7;25,26), said front cover (6;25) comprising an air-permeable material.

20. The method of claim 19, characterized in that said front cover (6;25) comprises a woven screen.

## Patentansprüche

1. Leichte Explosionsschutzmatte (10; 17) zum Schutz gegen die zerstörerische Wucht einer Explosion,
wobei die Matte (10; 17) eine erste Schicht (3; 18) aus Streckgitternetz, eine zweite von der ersten Schicht (3; 18) beabstandete Schicht (4; 20) aus Streckgitternetz und eine innere Kernschicht (5; 22,23) aus luftdurchlässigem Material, welche die erste und zweite Schicht trennt, aufweist,
dadurch gekennzeichnet, daß die erste und zweite Schicht (3, 4; 18, 20) aus Streckgitternetz aus geschlitzter Metallfolie (10) mit einer Dicke in dem Bereich von etwa 0,028 bis 1,0 mm hergestellt werden, und die innere Kernschicht (5; 22,23) wenigstens 2,54 cm (1 Inch) dick ist.

2. Explosionsschutzmatte gemäß Anspruch 1, dadurch gekennzeichnet, daß das Streckgitternetz aus Magnesiumslegierungsfolie (40) hergestellt ist.

3. Explosionsschutzmatte gemäß Anspruch 2, dadurch gekennzeichnet, daß die Magnesiumlegierungsfolie (4) eine Dicke in dem Bereich von ungefähr 0,028 bis 0,5 mm hat.

4. Explosionsschutzmatte gemäß Anspruch 1, dadurch gekennzeichnet, daß das Streckgitternetz eine Dicke von ungefähr 2 bis 8 mm in seiner gestreckten Form hat.

5. Explosionsschutzmatte gemäß Anspruch 1, dadurch gekennzeichnet, daß der innere Kern (5; 22, 23) aus luftdurchlässigem Material Glasfasern aufweist.

6. Explosionsschutzmatte gemäß Anspruch 1, dadurch gekennzeichnet, daß der innere Kern (5; 22, 23) aus luftdurchlässigem Material Baumwolle in Lagen aufweist.

7. Explosionsschutzmatte gemäß Anspruch 1, dadurch gekennzeichnet, daß der innere Kern (22, 23) aus luftdurchlässigem Material eine Anordnung von aus Streckgitternetz gebildeten Kugeln (22, 23) aufweist.

8. Explosionsschutzmatte gemäß Anspruch 1, dadurch gekennzeichnet, daß die Matte (10; 17) zwischen einer vorderen und hinteren Abdeckung (6, 7; 25, 26) gehalten wird, wobei die vordere Abdeckung (6; 25) ein luftdurchlässiges Material aufweist.

9. Explosionsschutzmatte gemäß Anspruch 8, dadurch gekennzeichnet, daß die vordere Abdeckung (6; 25) ein gewebtes Gitter aufweist.

10. Explosionsschutzmatte gemäß Anspruch 1, dadurch gekennzeichnet, daß die Komponentschichten der Matte (17) miteinander verbunden sind, um eine Verbundstruktur zu bilden mit:
a. einer vorderen Abdeckschicht (25) aus einem luftdurchlässigen Material,
b. einer ersten Explosionsschutzschicht mit der ersten Schicht (18) und wenigstens einer weiteren Schicht aus Streckgitternetz, welche in Kontakt miteinander und angrenzend an die vordere Abdeckschicht (25) angeordnet sind, wobei die weitere Schicht aus Streckgitternetz in der ersten Explosionsschutzschicht aus geschlitzter Metallfolie mit einer Dicke in dem Bereich von ungefähr 0,028 bis 1,0 mm hergestellt ist;
c. einer zweiten Explosionsschutzschicht mit wenigstens einer zweiten Schicht (20) aus Streckgitternetz, wobei die zweite Explosionsschutzschicht von der ersten Explosionsschutzschicht beabstandet ist;
d. der inneren Kernschicht (22, 23) aus luftdurchlässigem Material, welche die erste und zweite Explosionsschutzschicht trennt; und
e. einer hinteren Abdeckschicht (26) welche mit der vorderen Abdeckschicht (25) verbunden ist und mit ihr zusammenwirkt, um die Verbundstruktur bereitzustellen.

11. Explosionsschutzmatte gemäß Anspruch 10, dadurch gekennzeichnet, daß sie eine zweite innere Kernschicht (24) aus luftdurchlässigem Material von wenigstens 2,54 cm (1 Inch) Dicke aufweist, welche die zweite Explosionsschutzschicht und die hintere Abdeckschicht (26) trennt.

12. Verfahren zum Schutz eines Bauteils gegen die zerstörerische Wucht eines Sprengstoffs, mit:
Anordnung einer schichtförmigen, leichten Explosionsschutzmatte (10; 17) zwischen dem Bauteil und dem Sprengstoff, welche eine erste Schicht (3; 18) aus porösem Gitternetz, eine zweite von der ersten Schicht (3; 18) beabstandete Schicht (4; 20) aus porösem Gitternetz und eine innere Kernschicht (5; 22, 23) aus luftdurchlässigem Material, welche die erste und zweite Schicht trennt, aufweist,
dadurch gekennzeichnet, daß die erste und zweite Schicht (3, 4; 18, 20) Streckgitternetz aus geschlitzter Metallfolie (10) mit einer Dicke in dem Bereich von ungefähr 0,028 bis 1,0 mm hergestellt werden,
wobei die zwischen die erste und zweite Schicht gelegte innere Kernschicht (5; 22, 23) wenigstens 2,54 cm (1 Inch) dick ist.

13. Verfahren gemäß Anspruch 12, dadurch gekennzeichnet, daß das Streckgitternetz aus Magnesiumlegierungsfolie hergestellt ist.

14. Verfahren gemäß Anspruch 13, dadurch gekennzeichnet, daß die Magnesiumlegierungsfolie (40) eine Dicke in dem Bereich von 0,028 bis 0,5 mm aufweist.

15. Verfahren gemäß Anspruch 12, dadurch gekennzeichnet, daß die Magnesiumlegierungsfolie eine Dicke von ungefähr 2 bis 8 mm in ihrer gestreckten Form aufweist.

16. Verfahren gemäß Anspruch 12, dadurch gekennzeichnet, daß der innere Kern (5; 22, 23) aus luftdurchlässigem Material Glasfasern aufweist.

17. Verfahren gemäß Anspruch 12, dadurch gekennzeichnet, daß der innere Kern (5; 22, 23) aus luftdurchlässigem Material Baumwolle in Lagen aufweist.

18. Verfahren gemäß Anspruch 12, dadurch gekennzeichnet, daß der innere Kern (22, 23) aus luftdurchlässigem Material eine Anordnung von aus Streckgitternetz gebildeten Kugeln (22, 23) aufweist.

19. Verfahren gemäß Anspruch 12, dadurch gekennzeichnet, daß die Matte (10; 17) zwischen einer vorderen und hinteren Abdeckung (6, 7; 25, 26) gehalten wird, wobei die vordere Abdeckung (6; 25) ein luftdurchlässiges Material aufweist.

20. Verfahren gemäß Anspruch 19, dadurch gekennzeichnet, daß die vordere Abdeckung (6; 25) ein gewebtes Gitter aufweist.

## Revendications

1. Rembourrage antidéflagrant de peu de poids (10; 17) à des fins de protection contre l'impact destructif d'une explosion,
ledit rembourrage (10; 17) comprenant une première feuille (3; 18) d'un treillis métallique expansé, une seconde feuille (4; 20) d'un treillis métallique expansé espacé de ladite première feuille (3; 18), et une couche centrale interne (5; 22, 23) d'une matière perméable à l'air séparant lesdites première et seconde feuilles,
caractérisé en ce que lesdites première et seconde feuilles (3, 4; 18, 20) de treillis métallique expansé sont réalisées à partir d'une mince feuille métallique refendue (10) possédant une épaisseur dans le domaine d'environ 0,028 à 1,0 mm, et
en ce que ladite couche centrale interne (5; 22, 23) possède une épaisseur d'au moins 2,54 cm (1 pouce).

2. Rembourrage antidéflagrant selon la revendication 1, caractérisé en ce que ledit treillis métallique expansé est réalisé à partir d'une mince feuille d'alliage de magnésium (40).

3. Rembourrage antidéflagrant selon la revendication 2, caractérisé en ce que ladite mince feuille d'alliage de magnésium (4) possède une épaisseur dans le domaine d'environ 0,028 à 0,5 mm.

4. Rembourrage antidéflagrant selon la revendication 1, caractérisé en ce que ledit treillis métallique expansé possède une épaisseur d'environ 2 à 8 mm dans sa forme expansée.

5. Rembourrage antidéflagrant selon la revendication 1, caractérisé en ce que ladite partie centrale interne (5; 22, 23) constituée d'une matière perméable à l'air comprend des fibres de verre.

6. Rembourrage antidéflagrant selon la revendication 1, caractérisé en ce que ladite partie centrale interne (5; 22, 23) constituée d'une matière perméable à l'air comprend une nappe d'ouate.

7. Rembourrage antidéflagrant selon la revendication 1, caractérisé en ce que ladite partie centrale interne (22, 23) constituée d'une matière perméable à l'air comprend un assemblage de billes (22, 23) formé à partir d'un treillis métallique expansé.

8. Rembourrage antidéflagrant selon la revendication 1, caractérisé en ce que ledit rembourrage (10; 17) est retenu entre des recouvrements frontal et dorsal (6, 7; 25, 26), ledit recouvrement frontal (6; 25) comprenant une matière perméable à l'air.

9. Rembourrage antidéflagrant selon la revendication 8, caractérisé en ce que ledit recouvrement frontal (6; 25) comprend un pare-éclats en treillis.

10. Rembourrage antidéflagrant selon la revendication 1, caractérisé en ce que les couches qui composent ledit rembourrage (17) sont liées l'une à l'autre pour former une structure unifiée contenant:
a. une couche de recouvrement frontale (25) constituée d'une matière perméable à l'air,
b. une première couche antidéflagrante comprenant ladite première feuille (18) et au moins une feuille supplémentaire constituée dudit treillis métallique expansé, déposées en contact l'une avec l'autre et en position adjacente à ladite couche de recouvrement frontale (25), ladite feuille supplémentaire constituée d'un treillis métallique expansé dans ladite première couche antidéflagrante étant constituée d'une mince feuille métallique refendue possédant une épaisseur dans le domaine d'environ 0,028 à 1,0 mm;
c. une seconde couche antidéflagrante comprenant au moins ladite seconde feuille (20) constituée d'un treillis métallique expansé, ladite seconde couche antidéflagrante étant espacée de ladite première couche antidéflagrante;
d. ladite couche centrale interne (22, 23) constituée d'une matière perméable à l'air séparant lesdites première et seconde couches antidéflagrantes; et
e. une couche de recouvrement dorsale (26) liée à et coopérant avec ladite couche de recouvrement frontale (25) pour procurer ladite structure unifiée.

11. Rembourrage antidéflagrant selon la revendication 10, caractérisé en ce qu'il comprend une seconde couche centrale interne (24) constituée d'une matière perméable à l'air possédant une épaisseur d'au moins 2,54 cm (1 pouce) séparant ladite seconde couche antidéflagrante et ladite couche de recouvrement dorsale (26).

12. Procédé de protection d'une structure contre l'impact destructif d'un explosif, comprenant le fait de:
intercaler entre ladite structure et ledit explosif un rembourrage antidéflagrant stratifié de peu de poids (10; 17) comprenant une première feuille (3-18) constituée d'un treillis métallique poreux, une seconde feuille (4; 20) constituée d'un treillis métallique poreux possédant ladite première feuille (3; 18), et une couche centrale interne (5; 22, 23) constituée d'une matière perméable à l'air séparant lesdites première et seconde feuilles,
caractérisé par le fait que l'on prépare lesdites première et seconde feuilles (3, 4, 18, 20) constituées d'un treillis métallique expansé à partir d'une mince feuille métallique refendue (10) possédant une épaisseur dans le domaine d'environ 0,028 à 1,0 mm,
ladite couche centrale interne (5; 22, 23) intercalée entre lesdites première et seconde feuilles possède une épaisseur d'au moins 2,54 cm (1 pouce).

13. Procédé selon la revendication 12, caractérisé en ce que ledit treillis métallique expansé est réalisé à partir d'une mince feuille d'alliage de magnésium.

14. Procédé selon la revendication 13, caractérisé en ce que ladite mince feuille d'alliage de magnésium (40) possède une épaisseur dans le domaine d'environ 0,028 à 0,5 mm.

15. Procédé selon la revendication 12, caractérisé en ce que ledit treillis métallique expansé possède une épaisseur d'environ 2 à 8 mm dans sa forme expansée.

16. Procédé selon la revendication 12, caractérisé en ce que ladite partie centrale interne (5; 22, 23) constituée d'une matière perméable à l'air comprend des fibres de verre.

17. Procédé selon la revendication 12, caractérisé en ce que ladite partie centrale interne (5; 22, 23) constituée d'une matière perméable à l'air comprend une nappe d'ouate.

18. Procédé selon la revendication 12, caractérisé en ce que ladite partie centrale interne (22, 23) constituée d'une matière perméable à l'air comprend un assemblage de billes (22, 23) formé à partir d'un treillis métallique expansé.

19. Procédé selon la revendication 12, caractérisé en ce que ledit rembourrage (10; 17) est retenu entre des recouvrements frontal et dorsal (6, 7; 25, 26), ledit recouvrement frontal (6; 25) comprenant une matière perméable à l'air.

20. Procédé selon la revendication 19, caractérisé en ce que ledit recouvrement frontal (6; 25) comprend un pare-éclats en treillis.
